# EUROPÄISCHE PATENTSCHRIFT

(11) **EP 2 544 582 B1**
(45) Veröffentlichungstag und Bekanntmachung des Hinweises auf die Patenterteilung: **22.02.2017**
(21) Anmeldenummer: 10779244.2
(22) Anmeldetag: 06.11.2010
(51) Int. Cl.: A61B 5/145, A61B 5/1486, G01N 27/327, H01M 8/16, C12Q 1/00

(54) **Verfahren zur elektrochemischen Messung einer Analytkonzentration in-vivo und Brennstoffzelle hierfür**
Method for the electrochemical measurement of an analyte concentration in vivo, and fuel cell for this purpose
Procédé pour la mesure électrochimique d'une concentration en analyte in vivo et pile à combustible à cet effet

(30) Priorität: 11.03.2010 EP 10002588
(43) Veröffentlichungstag der Anmeldung: 16.01.2013
(73) Patentinhaber: Roche Diabetes Care GmbH, 68305 Mannheim (DE); F. Hoffmann-La Roche AG, 4070 Basel (CH)
(72) Erfinder: OCVIRK, Gregor, 55283 Nierstein (DE); KÖLKER, Karl-Heinz, 67269 Grünstadt (DE)
(74) Vertreter: Twelmeier Mommer & Partner
(86) Internationale Anmeldenummer: PCT/EP2010/006770
(87) Internationale Veröffentlichungsnummer: WO 2011/110202

(56) Entgegenhaltungen:
- EP-A2- 0 300 082
- WO-A1-97/19344
- WO-A1-03/019170
- US-A1- 2005 118 494
- PIZZARIELLO A ET AL: "A glucose/hydrogen peroxide biofuel cell that uses oxidase and peroxidase as catalysts by composite bulk-modified bioelectrodes based on a solid binding matrix.", 15. Mai 2002 (2002-05-15), BIOELECTROCHEMISTRY (AMSTERDAM, NETHERLANDS) 15 MAY 2002 LNKD- PUBMED:12009453, VOL. 56, NR. 1-2, PAGE(S) 99 - 105, XP002592399, ISSN: 1567-5394 ExperimentalAbbildung 1

## Beschreibung

Die Erfindung geht aus von einem Verfahren mit den im Oberbegriff des Anspruchs 1 angegebenen Merkmalen, wie es beispielsweise aus der WO 03/019170 A1 bekannt ist.

Aus der US 3837339 ist es bekannt, Brennstoffzellen als elektrochemische Sensoren zur in-vivo Messung von Analytkonzentrationen, beispielsweise der Glucosekonzentration, zu verwenden. Der zu messende Analyt wird dabei als Brennstoff der Brennzoffzelle katalytisch umgesetzt. Die in der US 3837339 beschriebene Brennstoffzelle oxidiert an der Anode Glucose und erzeugt dabei Gluconsäure. An der Kathode wird Sauerstoff reduziert.

Um die Effizienz dieser Reaktionen zu verbessern, ist es bekannt, die Kathode mit einer Enzymschicht zu versehen, die ein Enzym zur katalytischen Umsetzung des Analyten enthält und auf die Anode eine Enzymschicht mit einem Enzym zur katalytischen Reduktion von Sauerstoff aufzubringen. Derartige Brennstoffzellen sind beispielsweise aus der US 2005/0118494 A1 bekannt. Bei diesen Brennstoffzellen ist die Anode mit einer Enzymschicht bedeckt, die Glucoseoxidase enthält, und die Kathode mit einer Enzymschicht, die Laccase enthält. Auf diese Weise können die Anodenreaktion, also Oxidation von Glucose, und die Kathodenreaktion, also die Reduktion von Sauerstoff, beschleunigt werden.

Ein Problem bei der in-vivo Messung von Analytkonzentrationen mit derartigen Sensoren ist, dass die Intensität des Messsignals durch Sauerstoffmangel in der Umgebung der Kathode beeinträchtigt werden kann. Insbesondere bei längerem Betrieb kann in der Umgebung der Kathode eine Verarmung der Sauerstoffkonzentration eintreten, die in subkutanem Fettgewebe ohnehin großen Schwankungen unterliegt und deshalb das Messsignal verfälschen kann. Ein weiteres Problem ist, dass bei der katalytischen Umsetzung von Analyten häufig gesundheitlich bedenkliche oder sogar giftige Zwischenprodukte entstehen, beispielsweise Wasserstoffperoxid. Treten derartige Zwischenprodukte aus dem Sensor aus, kann dies zu Entzündungen führen und ein vorzeitiges Entfernen des Sensors erforderlich machen.

Aufgabe der vorliegenden Erfindung ist es, einen Weg aufzuzeigen, wie sich kostengünstig Analytkonzentrationen präzise über längere Zeit messen lassen

Diese Aufgabe wird durch ein Verfahren mit den im Anspruch 1 angegebenen Merkmalen und durch eine Brennstoffzelle gemäß Anspruch 4 gelöst. Vorteilhafte Weiterbildungen der Erfindung sind Gegenstand von Unteransprüchen.

Erfindungsgemäß wird in der Enzymschicht durch katalytische Umsetzung des zu messenden Analyten ein Produkt erzeugt, das dann an der Anode oxidiert und an der Kathode reduziert wird. Auf diese Weise lässt sich erreichen, dass das Messsignal von der lokalen Sauerstoffkonzentration unabhängig ist, da die im Stand der Technik gebräuchliche Reduktionsreaktion von Sauerstoff an der Kathode nicht mehr benötigt wird. Mit dem erfindungsgemäßen Verfahren lassen sich deshalb Analytkonzentrationen in-vivo auch über längere Zeit mit einer verbesserten Genauigkeit messen.

Bei dem erfindungsgemäßen Verfahren kann durch katalytische Umsetzung des zu messenden Analyten beispielsweise Wasserstoffperoxid als Produkt erzeugt werden, das anschließend an der Anode oxidiert und an der Kathode reduziert werden kann. Indem das gesundheitlich problematische Wasserstoffperoxid sowohl an der Anode als auch an der Kathode abgebaut wird, ist dessen Konzentration typischerweise nur etwa halb so groß wie bei herkömmlichen Sensoren, bei denen Wasserstoffperoxid nur an einer Elektrode umgesetzt wird. Schädliche Auswirkungen des Wasserstoffperoxids lassen sich deshalb weit besser vermeiden, als dies bei herkömmlichen Sensoren der Fall ist. Als Alternative zu Wasserstoffperoxid können auch andere Redoxamphotere wie Sulfite und Aldehyde verwendet werden. Als Beispiel für ein redoxamphoteres Aldehyd ist 5-Hydroxyindolylacetaldehyd zu nennen, welches aus Serotonin unter Verwendung von Monoaminoxidasen erzeugt werden kann und sowohl zur 5-Hydroxyindolyessigsäure oxidiert, als auch zum 5-Hydroxytryptophol reduziert werden kann.

Für das erfindungsgemäße Verfahren wird ein elektrochemischer Sensor zur in-vivo Messung einer Analytkonzentration verwendet, der eine Anode und eine Kathode, deren Oberfläche aus einem anderen Material als die Anode ist, aufweist, wobei die Anode und die Kathode zusammen mit einer Enzymschicht, die ein Enzym zur katalytischen Umsetzung des zu messenden Analyten enthält, eine Brennstoffzelle bilden. Die Enzymschicht des erfindungsgemäßen Sensors bildet einen Diffusionspfad für ein durch katalytische Umsetzung des Analyten erzeugtes Produkt, so dass der Sensor dieses Produkt an der Anode oxidiert und an der Kathode reduziert.

Als Konkurrenzreaktion zur Reduktion des durch katalytische Umsetzung des Analyten erzeugten Produkts kommt an der Kathode naturgemäß die Reduktion von Sauerstoff in Frage. Diese Konkurrenzreaktion ist aber praktisch vernachlässigbar. Die Reduktion des durch katalytische Umsetzung des Analyten erzeugten Produkts überwiegt. Bei Wasserstoffperoxid als Produkt beträgt die Reaktionsrate der Reduktion von Sauerstoff typischer Weise weniger als ein Zehntel, in der Regel sogar weniger als ein Hundertstel, der Reaktionsrate der Reduktion von Wasserstoffperoxid. Für 1 Sauerstoffmolekül werden also mehr als 10, in der Regel sogar mehr als 100, Wasserstoffperoxidmoleküle reduziert. Bei einem erfindungsgemäßen Sensor wird das Produkt also als signalbestimmende Reaktion an der Anode oxidiert und an der Kathode reduziert. Dies bedeutet, dass die Signalbeiträge von eventuellen Konkurenzreaktionen weniger als 10% der Signalintensität ausmachen.

Die Vernachlässigbarkeit der Sauerstoffreduktion beruht zum einen auf einer in Redox-Systemen stets mehr oder weniger stark auftretenden Überspannung des Sauerstoffs. Die Reduktionsreaktion von Sauerstoff ist durch diese Überspannung gehemmt und findet deshalb wesentlich langsamer statt als die Reduktion des durch katalytische Umsetzung des Analyten erzeugten Produkts. Zum anderen ist das Normalpotential von Sauerstoff mit nur 1,22 V relativ klein, insbesondere im Vergleich zu dem Normalpotential von Wasserstoffperoxid von 1,77 V, so dass die Reduktion von Sauerstoff auch in dieser Hinsicht thermodynamisch benachteiligt ist. Eine erfindungsgemäße Brennstoffzelle lässt sich nicht nur als Sensor zur Messung einer Analytkonzentration verwenden, sondern kann im Körper eines Patienten auch als Energiequelle einer implantierten Vorrichtung verwendet werden, beispielsweise für einen Herzschrittmacher.

Bei einer erfindungsgemäßen Brennstoffzelle bildet die Enzymschicht einen Diffusionspfad für das durch katalytische Umsetzung des Stoffes bzw. des Analyten erzeugte Produkt, so dass dieses Produkt bei Gebrauch des Sensor an der Anode oxidiert und an der Kathode reduziert wird. Dies lässt sich im einfachsten dadurch erreichen, dass die Enzymschicht sowohl an der Anode als auch an der Kathode anliegt. Beispielsweise können die Anode und die Kathode auf einem gemeinsamen Träger angeordnet sein, wobei die Enzymschicht sowohl die Anode als auch die Kathode bedeckt. Eine andere Möglichkeit ist es, die Enzymschicht zwischen der Anode und der Kathode anzuordnen. Insbesondere bei einer sandwichartigen Anordnung können zwischen der Enzymschicht und der Anode oder zwischen der Enzymschicht und der Kathode weitere Zwischenschichten vorgesehen sein, sofern diese für das redoxamphotere Produkt, beispielsweise Wasserstoffperoxid, durchlässig sind. Soweit im Folgenden von einem erfindungsgemäßen Sensor die Rede ist, lassen sich die dabei erwähnten Merkmale auch bei einer erfindungsgemäßen Brennstoffzelle nutzen, die als Energiequelle eines Herzschrittmachers oder einer anderen Vorrichtung im Körper eines Patienten verwendet wird. Insbesondere lässt sich ein erfindungsgemäßer Sensor ohne Weiteres als Energiequelle für einen Herzschrittmacher verwenden.

Ein erfindungsgemäßer Sensor lässt sich sehr einfach und kostengünstig herstellen, da im Prinzip nur eine Anode, eine Kathode und die Enzymschicht benötigt werden. Der mit dem Herstellen separater Enzymschichten für Anode und Kathode verbundene Aufwand lässt sich bei einem erfindungsgemäßen Sensor vermeiden.

Die in der Enzymschicht eingelagerten Enzymmoleküle sind bevorzugt eine Oxidase, beispielsweise eine Glucoseoxidase oder Lactoseoxidase, können aber beispielsweise auch eine Hydrogenase sein. Die Enzymmoleküle können dabei in der Enzymschicht kovalent gebunden sein. Möglich ist es aber auch, dass die Enzymmoleküle dem Material der Enzymschicht lediglich zugemischt sind und in der Enzymschicht beweglich sind. Insbesondere in diesem Fall ist die Enzymschicht bevorzugt mit einer Deckschicht, beispielsweise einem Polymerfilm bedeckt, der für Enzymmoleküle undurchlässig ist. Geeignete Deckschichten, die für Wasser und die zu messenden Analytmoleküle durchlässig sind, können beispielsweise aus Polyurethanen, Polyvinylchloriden, Polycarbonaten, Polytetrafluorethylen, Acrylaten oder Silikonen hergestellt werden. Geeignet sind insbesondere sulfonierte Tetrafluorethylene, wie sie unter der Marke Nafion im Handel erhältlich sind.

Für die Enzymschicht können insbesondere wasserdurchlässige Polymere verwendet werden. Geeignet sind insbesondere Kunststoffe, beispielsweise Polyurethan. Eine weitere Möglichkeit sind beispielsweise Pektin, Gelatine oder andere wasserdurchlässigen Naturstoffe. Bevorzugt ist die Enzymschicht als eine Kunststoffmatrix mit eingelagerten Enzymmolekülen ausgebildet.

Die Enzymschicht besteht idealer Weise aus Material, das im getrockneten Zustand ein Isolator ist. Zum Betrieb des Sensors nimmt die Enzymschicht Wasser auf und wird zu einem Ionenleiter. Eine hohe Beweglichkeit des Zwischenprodukts innerhalb der Enzymschicht ist wünschenswert.

Die Kathode des erfindungsgemäßen Sensors hat bevorzugt eine metallische Oberfläche, beispielsweise aus Palladium. Geeignet sind aber auch andere Edelmetalle und Edelmetalllegierungen. Die Kathode kann auf diese Weise kostengünstig als Leiterbahn auf einem Träger aus Kunststoff ausgebildet sein. Die Kathode kann auch mit einer nicht metallischen Oberfläche ausgeführt werden, die eine Reduktion des Zwischenproduktes zulässt.

Die Anode kann ebenfalls eine metallische Oberfläche haben, wenn diese aus einem anderen Metall als die Kathodenoberfläche besteht. Bevorzugt hat die Anode aber eine nicht metallische Oberfläche, beispielsweise eine kohlenstoffpartikelhaltige oder graphitpartikelhaltige Oberfläche. Die nicht metallische Oberfläche der Anode ist bevorzugt als Belag auf einem metallischen Leiter angeordnet.

Ruß- oder Graphitpartikel lassen sich mit einem polymeren Bindemittel gut zu einer Paste mischen, in die zusätzliche katalytisch wirksame Partikel eingemischt werden können. Durch geeignete Einmischungen können Pasten unterschiedlicher Zusammensetzungen generiert werden, die dann der Ausbildung der Kathode und/oder Anode auf metallische Leiterbahnen dienen.

Bevorzugt ist ein zu der Anode führender Leiter über einen elektrischen Widerstand mit einem zu der Kathode führenden Leiter verbunden. Dieser Widerstand bewirkt einen kontinuierlichen Strom zwischen der Anode und der Kathode, so dass das redoxamphotere Produkt kontinuierlich abgebaut wird, was insbesondere bei gesundheitlich problematischen Produkten wie Wasserstoffperoxid ein wichtiger Vorteil ist.

Ein weiterer Vorteil der Erfindung besteht darin, dass im Gegensatz zu herkömmlichen amperometrischen Wasserstoffperoxid-Sensoren, keine externe Spannungsversorgung benötigt wird um das Wasserstoffperoxid kontinuierlich abzubauen.

Der elektrische Widerstand kann beispielsweise als Leiterbahn auf einem Träger ausgebildet sein, der auch den zu der Anode und den zu der Kathode führenden Leiter trägt. Die zur Ausbildung des Widerstands verwendete Leiterbahn kann aus dem selben Material wie die zu der Anode und der Kathode führenden Leiterbahnen sein, wobei deren Durchmesser, insbesondere deren Dicke und/oder deren Breite, entsprechend klein gewählt ist, um den gewünschten Wert des Widerstands zu erhalten. Bevorzugt ist der Widerstand aber aus einem anderen Material als die zu der Anode und der Kathode führenden Leiter. Beispielsweise kann eine Leiterbahn als eine Paste aus einem Widerstandmaterial auf den Träger aufgedruckt sein. Der Widerstand ist bevorzugt eine Materialmischung, die Kohlenstoffpartikel, beispielsweise Ruß und/oder Graphit, und Bindemittel enthält. Geeignetes Widerstandsmaterial kann beispielsweise eine kohlenstoffpartikelhaltige Paste sein, die sich nach dem Auftragen verfestigt. Der Widerstand hat bevorzugt einen Wert von mindestens 1 MOhm. Widerstandswerte von mehr als 1 GOhm sind in der Regel aber nicht vorteilhaft. Vorteilhaft sind in der Regel Widerstandswerte zwischen 10 MOhm und 100 MOhm.

Der elektrische Widerstand kann aber auch in einen Steckkontakt integriert werden, welcher während des Betriebes des Sensors mit beiden Leiterbahnenden leitend verbunden ist.

Eine vorteilhafte Weiterbildung der Erfindung sieht vor, dass sowohl die Reduktion als auch die Oxidation des redoxamphoteren Produktes, das durch enzymatische Umsetzung des zu messenden Anaylten erzeugt wird, mit elektrochemisch wirksamen Katalysatoren unterstützt werden. Geeignete Katalysatoren sind für die Oxidation von Wasserstoffperoxid beispielsweise Metalloxide, insbesondere Braunstein, und metallorganische Verbindungen, beispielsweise Kobalt-Phtalocyanin. Insbesondere Braunstein lässt sich als Pulver gut mit Graphitpartikeln mischen und auf diese Weise in die Oberfläche der Anode integrieren.

Weitere Einzelheiten und Vorteile der Erfindung werden an Ausführungsbeispielen unter Bezugnahme auf die beigefügten Zeichnungen erläutert. Gleiche und einander entsprechende Teile sind dabei mit übereinstimmenden Bezugszahlen bezeichnet. Es zeigen:
- Fig. 1: ein Ausführungsbeispiel eines erfindungsgemäßen Sensors;
- Fig. 2: eine Schnittansicht zu Figur 1;
- Fig. 3: ein Beispiel des Verlaufs der mit dem Sensor gemessenen Spannung in mV (linke Achse) sowie der Wasserstoffperoxidkonzentration in mMol (rechte Achse) über der Zeit;
- Fig. 4: ein Messbeispiel der von dem Sensor gelieferten Spannung in mV in Abhängigkeit von der Glucosekonzentration in mMol;
- Fig. 5: ein weiteres Ausführungsbeispiel eines erfindungsgemäßen Sensors;
- Fig. 6: eine Schnittdarstellung des in Figur 5 dargestellten Sensors;
- Fig. 7: ein weiteres Ausführungsbeispiel eines erfindungsgemäßen Sensors;
- Fig. 8: ein weiteres Ausführungsbeispiel eines erfindungsgemäßen Sensors in einem Längsschnitt;
- Fig. 9: ein weiteres Ausführungsbeispiel eines erfindungsgemäßen Sensors in einem Längsschnitt
- Fig. 10: eine Schnittansicht zu Figur 9 entlang der Schnittlinie AA;
- Fig. 11: eine Schnittansicht zur Figur 9 entlang der Schnittlinie BB;
- Fig. 12: ein weiteres Ausführungsbeispiel eines erfindungsgemäßen Sensors in einem Längsschnitt
- Fig. 13: eine Schnittansicht zu Figur 12 entlang der Schnittlinie AA;
- Fig. 14: eine Schnittansicht zu Figur 12 entlang der Schnittlinie BB;
- Fig. 15: eine Schnittansicht zu Figur 12 entlang der Schnittlinie CC;
- Fig. 16: ein weiteres Ausführungsbeispiel eines erfindungsgemäßen Sensors;
- Fig. 17: eine Schnittansicht zu Figur 16; und
- Fig. 18: ein weiteres Ausführungsbeispiel eines erfindungsgemäßen Sensors.

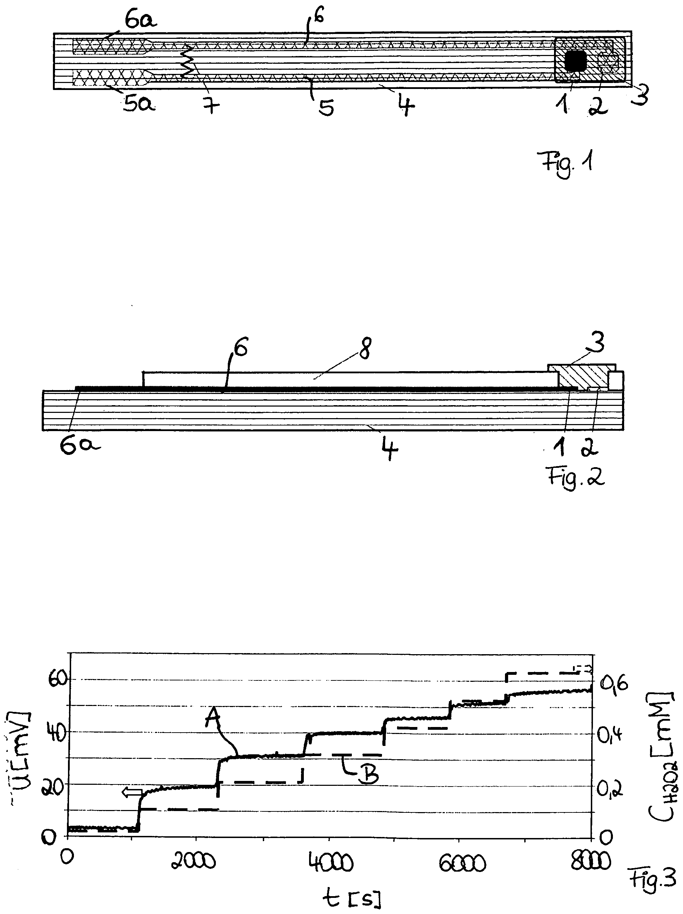

Der in Figur 1 dargestellte Sensor arbeitet nach dem Prinzip einer Brennstoffzelle. Aus dem zu messenden Analyten, beispielsweise Glucose oder Lactat, wird ein redoxamphoteres Produkt erzeugt, das den Brennstoff der Brennstoffzelle bildet. Die von der Brennstoffzelle gelieferte Energie ist deshalb umso größer, je größer die zu messende Analytkonzentration ist. Der elektrische Spannungsabfall über einen Lastwiderstand zwischen Anode und Kathode kann deshalb als Messsignal zur Bestimmung der Analytkonzentration verwendet werden.

Der in Figur 1 in einer Draufsicht und in Figur 2 in einem Längsschnitt dargestellte Sensor hat eine Anode 1 und eine Kathode 2, die von einer gemeinsamen Enzymschicht 3 bedeckt sind. Die Anode 1 und die Kathode 2 befinden sich jeweils am Ende einer auf einem Träger 4, beispielsweise einem Kunststoffblättchen, angeordneten Leiterbahn 5, 6. Die Leiterbahnen 5, 6 können aus Edelmetall, beispielsweise Palladium, bestehen, das auch die Oberfläche der Kathode 2 bilden kann. Die Anode 1 kann als Belag der Leiterbahn 5 ausgebildet sein, beispielsweise aus Kohlenstoffpartikeln und einem Bindemittel. Die beiden Leiterbahnen 5, 6 sind mit einem elektrischen Lastwiderstand 7 verbunden und einer wasserundurchlässigen, elektrisch isolierenden Schicht 8 bedeckt. Die Enden der Leiterbahnen 5, 6 ragen unter der Isolationsschicht 8 hervor. Die Kontaktfächen 5a, 6a sind deshalb ebenso wie die Anode 1 und die Kathode 2 nicht von der wasserundurchlässigen, elektrisch isolierenden Schicht 8 bedeckt.

Die in die wasserdurchlässige Enzymschicht 3 diffundierenden Analytmoleküle, beispielsweise Glucosemoleküle, werden durch die Enzymschicht 3 enthaltenden Enzymmoleküle, beispielsweise einer Oxidase, enzymatisch umgesetzt, wodurch ein redoxamphoteres Produkt, beispielsweise Wasserstoffperoxid, entsteht. Das redoxamphotere Produkt ist in der Enzymschicht 3 beweglich und gelangt deshalb sowohl zur Anode 1 als auch zur Kathode 2. An der Anode 1 wird das redoxamphotere Produkt oxidiert und an der Kathode 2 reduziert. Zur Förderung der Oxidationsreaktion kann dem Anodenmaterial ein Katalysator, beispielsweise Braunstein, zugemischt sein. Durch die elektrochemische Umsetzung des redoxamphoteren Produkts an Anode 1 und Kathode 2 entsteht eine elektrische Spannung zwischen Anode 1 und Kathode 2. Über den Lastwiderstand 7 wird die elektrische Spannung zwischen Anode 1 und Kathode 2 gemessen und als Messsignal zur Bestimmung der Analytkonzentration verwendet. Die Enden von der Anode 1 und der Kathode 2 abgewandten Enden 5a und 6a können verbreitert sein, um als Kontaktfelder das Anschließen eines Spannungsmessgeräts zu erleichtern.

Bei einer idealen Brennstoffzelle hängt die Spannung zwischen Anode 1 und Kathode 2 nur von den elektrochemischen Potentialen, die sich als Folge der Anoden- und der Kathodenreaktion einstellen und der Größe des Lastwiderstandes 7 zwischen Anode 1 und Kathode 2 ab. Die elektrische Spannung zwischen Anode 1 und Kathode 2 über den Lastwiderstand 7 stellt sich deshalb als Folge der Reaktionsraten der Reaktion an der Anode 1 und der Reaktion an der Kathode 2 ein.

Da bei dem dargestellten Sensor sowohl an der Anode 1 als auch an der Kathode 2 derselbe Stoff, bevorzugt Wasserstoffperoxid, umgesetzt wird, werden sowohl die Anodenreaktion als auch die Kathodenreaktion maßgeblich von der Analytkonzentration in der Enzymschicht 3 bestimmt. Die Rate, mit der das Produkt entsteht, das an der Anode 1 und Kathode 2 umgesetzt wird, ist nämlich der Analytkonzentration in einem weiten Konzentrationsbereich näherungsweise proportional.

Der Lastwiderstand 7 kann durch eine Leiterbahn aus Widerstandsmaterial, beispielsweise einer graphitpartikelhaltigen Paste, gebildet sein, welche die zu der Anode 1 führende Leiterbahn 5 mit der zu der Kathode 2 führende Leiterbahn 5 verbindet. Der Widerstand 7 ist bevorzugt unter der Isolationsschicht 8 angeordnet. Prinzipiell ist es aber auch möglich, mit dem Widerstand 7 die unter der Isolationsschicht 8 hervorragenden anschlussseitigen Enden der Leiterbahnen 5, 6 zu verbinden.

In Figur 3 ist ein Messbeispiel der von dem Sensor gelieferten Spannung in Millivolt zusammen mit der Wasserstoffperoxidkonzentration in Millimol über der Zeit t in Sekunden dargestellt. Die linke Ordinate gibt dabei die Spannung U in Millivolt für die Messkurve A und die rechte Ordinate die Wasserstoffperoxidkonzentration in Millimol für den dazugehörenden Konzentrationsverlauf, der durch die Kurve B dargestellt ist. Wie man sieht, stellt sich bei einem sprunghaften Anstieg der Wasserstoffperoxidkonzentration innerhalb von wenigen Sekunden eine neue Gleichgewichtsspannung zwischen Anode 1 und Kathode 2 über den Lastwiderstand 7 ein.

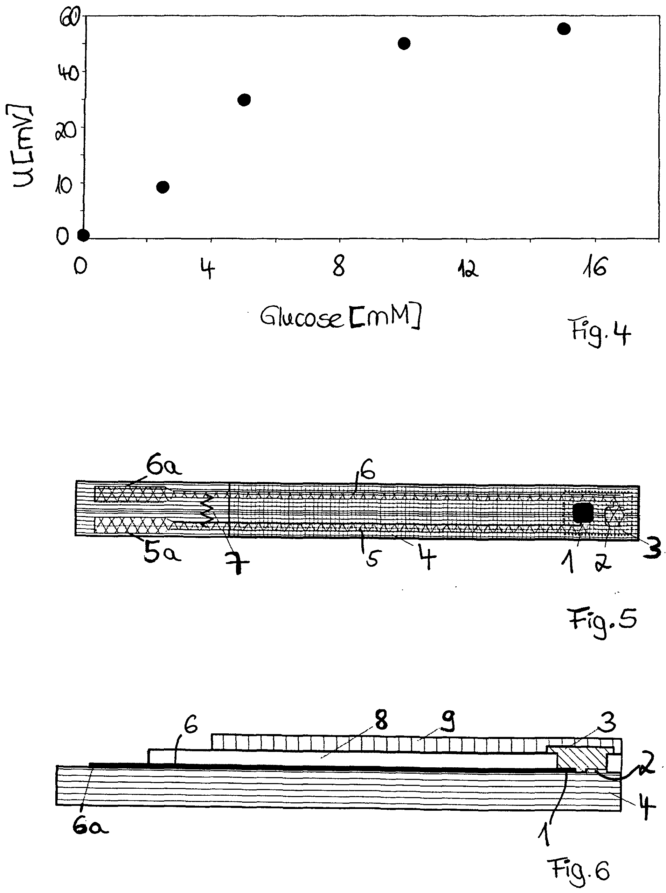

Figur 4 zeigt ein Messbeispiel der von dem Sensor gelieferten Spannung U in Millivolt in Abhängigkeit von der Glucosekonzentration in Millimol/Liter. Dabei ist zu erkennen, dass die elektrische Spannung zwischen Anode und Kathode umso größer ist, je größer die Glucosekonzentration ist. Aus der gemessenen Spannung kann deshalb die dazugehörende Glucosekonzentration mit einer Eichkurve bestimmt werden. Zur Bestimmung einer Analytkonzentration kann also insbesondere die an einem Widerstand 7 abfallende Spannung gemessen werden, der einen zu der Anode führenden Leiter 5 mit einem zu der Kathode führenden Leiter 6 verbindet.

Figur 5 zeigt ein weiteres Ausführungsbeispiel eines erfindungsgemäßen Sensors. Figur 6 zeigt einen Längsschnitt zur Figur 5. Der in den Figuren 5 und 6 dargestellte Sensor unterscheidet sich von dem in den Figuren 1 und 2 dargestellten Ausführungsbeispiel im Wesentlichen nur durch eine die Enzymschicht 3 bedeckende Deckschicht 9. Die Deckschicht 9 ist für Analytmoleküle sowie Wasser durchlässig und kann mehrere Funktionen erfüllen, die jeweils zu einer Verbesserung des Sensors führen, aber nicht unbedingt erforderlich sind.

Die Deckschicht 9 kann beispielsweise für Enzymmoleküle undurchlässig sein. Indem einem Austreten von Enzymmolekülen aus dem Sensor entgegengewirkt wird, lässt sich die Verträglichkeit des Sensors verbessern, da austretende Enzymmoleküle im Körpergewebe eines Patienten schädliche Auswirkungen haben könnten. Diese Funktion der Deckschicht 9 ist insbesondere dann vorteilhaft, wenn die Enzymmoleküle in der Enzymschicht 3 nicht kovalent gebunden sind. Die Enzymschicht 3 kann Enzymmoleküle kovalent gebunden enthalten. Beispielsweise können Enzymmoleküle kovalent mit Polymeren einer Matrix verbunden sein. Möglich ist es aber auch, dass die Enzymmoleküle dem Material der Enzymschicht 3 lediglich zumischt sind und darin diffundieren können. Insbesondere im letzteren Fall ist eine für Enzymmoleküle undurchlässige Deckschicht 9 ein wesentlicher Vorteil. Die Deckschicht 9 kann zu diesem Zweck beispielsweise aus Polyurethanen, Polyvinylchlorid, Polycarbonat, Polytetrafluorethylen, Polyacrylaten, Silikonen, Polyvinylpyrrol oder Mischungen derartiger Polymere hergestellt sein.

Die Deckschicht 9 kann vorteilhaft auch ein Reservoir für Analytmoleküle bilden, aus dem bei einer vorübergehenden Störung des Flüssigkeitsaustausches in der Umgebung des Sensors Analytmoleküle zu der Enzymschicht 3 gelangen können. Wird vorübergehend, beispielsweise durch Körperbewegungen des Patienten, der Austausch von Körperflüssigkeit in der Umgebung des Sensors eingeschränkt oder sogar unterbunden, können in der Deckschicht 9 gespeicherte Analytmoleküle weiterhin zu der Enzymschicht 3 diffundieren. Auf diese Weise kann die Deckschicht 9 bewirken, dass eine merkliche Verarmung der Analytkonzentration und eine entsprechende Verfälschung von Messergebnissen erst nach einer wesentlich größeren Zeitspanne auftreten. Die Deckschicht 9 kann zu diesem Zweck auch eine wesentliche größere Stärke haben, als dies in der nicht maßstäblichen Figur 6 den Eindruck erweckt.

Eine weitere Funktion der Deckschicht 9 kann darin bestehen, dem zu messenden Analyten einen Diffusionswiderstand entgegenzusetzen, also als Diffusionsbremse zu wirken. Die Deckschicht 9 bewirkt wegen ihres Diffusionswiderstands, dass pro Zeiteinheit weniger Analytmoleküle zu der Enzymschicht 3 gelangen. Durch die Deckschicht 9 kann deshalb die Rate, mit der Analytmoleküle umgesetzt werden, reduziert und somit einer Verarmung einer Analytkonzentration in der Umgebung des Sensors entgegengewirkt werden.

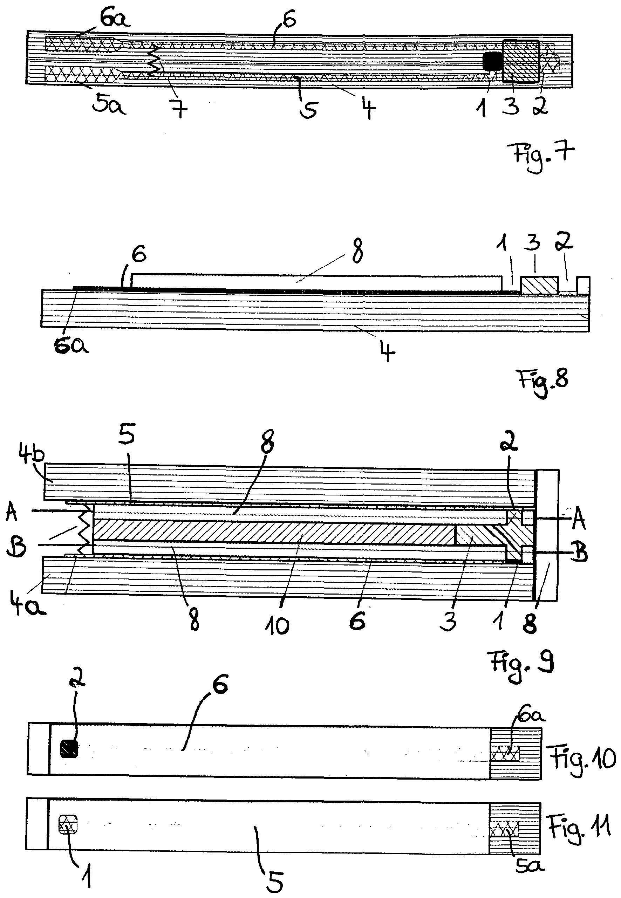

Ein weiteres Ausführungsbeispiel eines erfindungsgemäßen Sensors ist in Figur 7 dargestellt. Figur 8 zeigt einen Längsschnitt zu Figur 7. Dieses Ausführungsbeispiel unterscheidet sich von dem Ausführungsbeispiel der Figuren 1 und 2 im Wesentlichen nur dadurch, dass die Enzymschicht 3 die Anode 1 und die Kathode 2 nicht bedeckt. Stattdessen ist die Enzymschicht 3 zwischen der Anode 1 und der Kathode 2 angeordnet. Auch bei dieser Anordnung bildet die Enzymschicht 3 einen Diffusionspfad für den Analyten und das durch dessen Umsetzung gebildete redoxamphotere Produkt, beispielsweise Wasserstoffperoxid, zur Anode 1 und zur Kathode 2. Wie bei den übrigen Ausführungsbeispielen wird deshalb an der Anode 1 das Produkt oxidiert und an der Kathode 2 reduziert.

Das in den Figuren 7 und 8 dargestellte Ausführungsbeispiel kann mit einer Deckschicht 9 versehenen werden, wie dies vorstehend im Bezug auf das in den Figuren 5 und 6 dargestellte Ausführungsbeispiel erläutert wurde. Bevorzugt bedeckt eine eventuelle Deckschicht 9 bei dem Ausführungsbeispielen der Figuren 7 und 8 nicht nur die Enzymschicht 3, sondern auch die Anode 1 und die Kathode 2.

In Figur 9 ist ein weiteres Ausführungsbeispiel eines erfindungsgemäßen Sensors in einem Längsschnitt dargestellt. Figur 10 zeigt eine Schnittansicht zu Figur 9 entlang der Schnittlinie AA. Figur 11 eine Schnittlinie zu Figur 9 entlang der Schnittlinie BB.

Bei diesem Ausführungsbeispiel sind die Anode 1 und die Kathode 2 auf unterschiedlichen Trägern 4a, 4b angeordnet. Die Träger 4a, 4b können beispielsweise als Kunststoffplättchen ausgebildet sein und jeweils eine Leiterbahn 5, 6 tragen, an deren Enden sich die Anode 1 bzw. die Kathode 2 befinden. Die Enzymschicht 3 ist zwischen den beiden Trägern 4a, 4b angeordnet. Die Stirnseite dieser sandwichartigen Anordnung ist mit einer Deckschicht 9 bedeckt, wie sie im Zusammenhang mit dem Ausführungsbeispiel der Figuren 5 und 6 erläutert wurde. Die Leiterbahnen 5, 6 können ebenso wie bei den vorstehend beschriebenen Ausführungsbeispielen mit einer Isolationsschicht 8 bedeckt sein. Zusätzlich kann zwischen den beiden Trägern 4a, 4b ein Abstandhalter 10 angeordnet sein.

Der die beiden Leiterbahnen 5, 6 verbindende Lastwiderstand 7 kann bei diesem Ausführungsbeispiel ein separates Bauelement sein, das zwischen den Trägern 4a, 4b angeordnet ist. Beispielsweise kann dieses Bauelement mit den Leiterbahnen 5, 6 verlötet oder klemmend mit diesen verbunden sein. Möglich ist es insbesondere auch, dass der Abstandhalter 10 den Lastwiderstand 7 trägt. Beispielsweise kann das Widerstandselement 7 als leitfähige Paste in den Zwischenraum zwischen den beiden Trägern 4a, 4b auf die Stirnseite des Abstandhalters 10 aufgebracht werden, so dass der Lastwiderstand 7 die beiden Leiterbahnen 5, 6 kontaktiert.

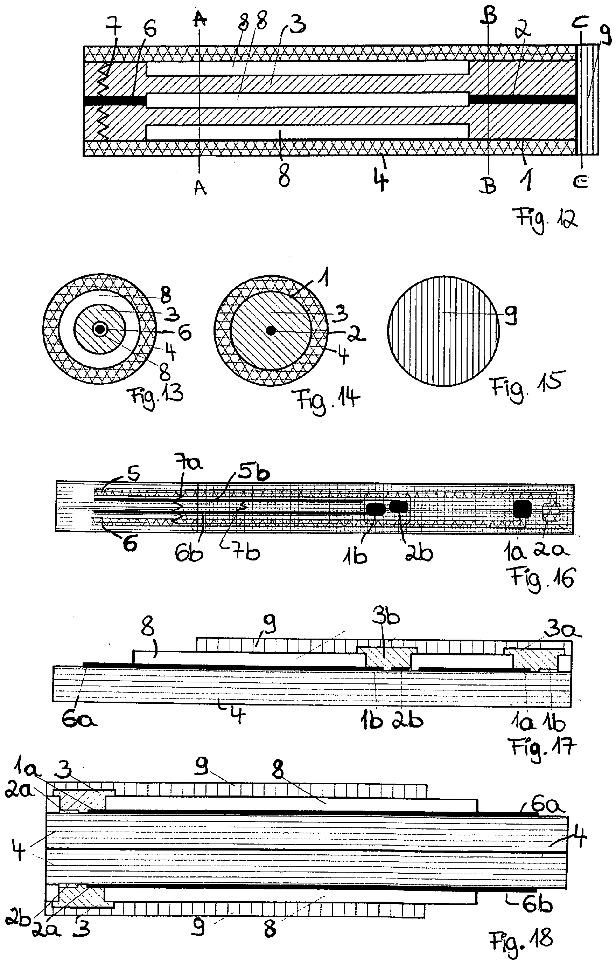

Ein weiteres Ausführungsbeispiel eines erfindungsgemäßen Sensors ist in Figur 12 in einem Längsschnitt dargestellt. Figur 13 zeigt einen Querschnitt dieses Sensors entlang der Figur 12 eingezeichneten Schnittlinie AA. Figur 14 zeigt einen Querschnitt dieses Sensors entlang der in Figur 12 eingezeichneten Schnittlinie BB. Figur 15 zeigt einen Querschnitt zu diesem Sensor entlang er in Figur 12 eingezeichneten Schnittlinie CC.

Bei diesem Ausführungsbeispiel ist die Anode 1 an der Innenseite einer Hülse angeordnet und die Kathode 2 an einem von der Hülse umgebenen Leiter. Dieses Ausführungsbeispiel kann dahingehend abgewandelt werden, dass die Kathode 2 an der Innenseite der Hülse und die Anode an dem von der Hülse umgebenden Leiter angeordnet ist. Der von der Hülse umgebene Leiter kann beispielsweise ein Draht sein. Die Hülse kann aus Metall ausgeführt sein oder ein Kunststoffröhrchen sein, dessen Innenseite metallisch beschichtet ist, um einen zu der an seiner Innenseite angeordneten Elektrode, bevorzugte Anode 1, führenden elektrischen Leiter auszubilden.

Ähnlich wie bei den vorstehend beschriebenen Ausführungsbeispielen sind die zu der Anode 1 und der Kathode 2 führenden Leiter 5, 6 mit einer Isolationsschicht 8 bedeckt. Zwischen der Anode 1 und der Kathode 2 befindet sich die Enzymschicht 3. Bei dem dargestellten Ausführungsbeispiel ist der verbleibende Innenraum des Sensors im Wesentlichen vollständig mit Enzymschicht 3, beispielsweise einer Enzymmoleküle enthaltenden Kunststoffmatrix gefüllt. Die Stirnseite des Sensors ist mit einer Deckschicht 9 bedeckt, wie sie auch bei dem vorstehend erläuterten Ausführungsbeispiel vorhanden sein kann.

Bei dem dargestellten Ausführungsbeispiel erstreckt sich die Enzymschicht 3 bis zu dem Lastwiderstand 7, der den Anodenleiter mit dem Kathodenleiter verbindet. Es kann jedoch vorteilhaft sein, einen Abschnitt der Hülse mit Isolationsmaterial aufzufüllen. Der Lastwiderstand 7 kann dann in einem Abstand von der Enzymschicht 3 angeordnet sein, beispielsweise auf dem die Hülse verschließenden Isolationsmaterial. Auch bei diesem Ausführungsbeispiel kann der Lastwiderstand 7 vorteilhaft als eine kohlenstoffpartikelhaltige Paste ausgebildet sein, die sich nach dem Auftragen verfestigt. Möglich ist es aber auch, den Lastwiderstand 7 als herkömmliches Widerstandselement auszubilden, das mit dem Anodenleiter und dem Kathodenleiter beispielsweise verlötet oder in anderer Weise elektrisch leitend verbunden ist.

In Figur 16 ist ein weiteres Ausführungsbeispiel einer erfindungsgemäßen Sensors dargestellt. Figur 17 zeigt eine Schnittansicht zu Figur 16. Dieses Ausführungsbeispiel unterscheidet sich von dem in den Figuren 5 und 6 dargestellten Ausführungsbeispiel im Wesentlichen nur dadurch, dass zwei Anodenkathodenpaare 1 a, 2a und 1b, 2b vorhanden sind. Zu der Anode 1a führt der Leiter 6, zu der Kathode 2a der Leiter 5, zu der Anode 1 b der Leiter 6b, zu der Kathode 2b der Leiter 5b.

Der dargestellte Sensor hat deshalb zwei Brennstoffzellen, so dass im Grunde zwei verschiedene Sensoren vorhanden sind. Die beiden Brennstoffzellen können zur Messung desselben Analyten vorgesehen sein oder durch Verwendung unterschiedlicher Enzyme zur Messung unterschiedlicher Analyten bestimmt sein. Beispielsweise kann eine der beiden Brennstoffzellen zur Messung von Glucose und die andere Brennstoffzelle zur Messung von Laktaten vorgesehen sein. Wenn beide Brennstoffzellen zur Messung desselben Analyts bestimmt sind, ist es besonders vorteilhaft, wenn die beiden Brennstoffzellen jeweils einen Sensor mit unterschiedlicher Sensitivität bilden. Indem niedrige Analytkonzentrationen mit der einen Brennstoffzelle und hohe Analytkonzentrationen mit der anderen Brennstoffzelle gemessen werden, lässt sich eine insgesamt höhere Messgenauigkeit erzielen. Unterschiedliche Messsensitivitäten lassen sich beispielsweise durch unterschiedliche Enzymkonzentrationen in den Enzymschichten realisieren. Eine weitere Möglichkeit ist es, die Enzymschichten mit unterschiedlich durchlässigen Deckschichten 9 zu bedecken.

Ein weiteres Ausführungsbeispiel eines erfindungsgemäßen Sensors ist in Figur 18 dargestellt. Ähnlich wie das in den Figuren 16 und 17 dargestellte Ausführungsbeispiel sind auch bei dem in Figur 18 dargestellten Sensor zwei unterschiedliche Brennstoffzellen vorhanden. Bei diesem Ausführungsbeispiel sind die beiden Brennstoffzellen jedoch nicht auf derselben Seite eines Substrats angeordnet. Stattdessen ist eine der beiden Brennstoffzellen auf der Oberseite und die andere Brennstoffzelle auf der Unterseite des Trägers 4 angeordnet.

### Bezugszahlen

- 1, 1a, 1b: Anode
- 2, 2a, 2b: Kathode
- 3: Enzymschicht
- 4: Träger
- 5, 5b: Leiter
- 5a: Kontaktfläche
- 6, 6b: Leiter
- 6a: Kontaktfläche
- 7: Lastwiderstand
- 8: Isolationsschicht
- 9: Deckschicht
- 10: Abstandshalter

## Patentansprüche

1. Verfahren zur elektrochemischen Messung einer Analytkonzentration in-vivo mit einer Brennstoffzelle, die den zu messenden Analyten mit einem in einer Enzymschicht (3) enthaltenen Enzym katalytisch umsetzt und eine von der zu messenden Analytkonzentration abhängende elektrische Spannung zwischen einer Anode (1, 1a, 1b) und einer Kathode (2, 2a, 2b) liefert und diese gemessen wird, **dadurch gekennzeichnet, dass** bei der katalytischen Umsetzung des zu messenden Analyten in der Enzymschicht (3) ein Produkt erzeugt wird, das als Brennstoff der Brennstoffzelle an der Anode (1, 1 a, 1 b) oxidiert und an der Kathode (2, 2a, 2b) reduziert wird.

2. Verfahren nach Anspruch 1, **dadurch gekennzeichnet, dass** die Oxidation des Produkts mit einem Katalysator, vorzugsweise einem Metalloxid oder einer metallorganische Verbindung, unterstützt wird.

3. Verfahren nach einem der vorstehenden Ansprüche, **dadurch gekennzeichnet, dass** die Anode (1, 1a, 1b) und die Kathode (2, 2a, 2b) über einen elektrischen Widerstand miteinander verbunden sind und als Messsignal der Analytkonzentration die elektrische Spannung zwischen der Anode (1, 1a, 1b) und der Kathode (2, 2a, 2b) als Spannungsabfall an dem Widerstand gemessen wird.

4. Brennstoffzelle mit einer Anode (1, 1 a, 1 b), einer Kathode (2, 2a, 2b), deren Oberfläche aus einem anderen Material als die Anode (1, 1a, 1b) ist, und einer Enzymschicht (3), die ein Enzym zur katalytischen Umsetzung eines im menschlichen Körper auftretenden Stoffes enthält, **dadurch gekennzeichnet, dass** die Enzymschicht (3) einen Diffusionspfad zur Anode und zur Kathode für ein durch katalytische Umsetzung des Stoffes erzeugtes Produkt bildet, so dass die Brennstoffzelle dieses Produkt an der Anode (1, 1a, 1b) oxidiert und an der Kathode (2, 2a, 2b) reduziert.

5. Elektrochemischer Sensor zur in-vivo Messung einer Analytkonzentration mittels einer Brennstoffzelle nach Anspruch 4, wobei der Stoff, zu dessen katalytischer Umsetzung die Enzymschicht das Enzym enthält, der zu messende Analyt ist.

6. Elektrochemischer Sensor nach Anspruch 5, **dadurch gekennzeichnet, dass** die Enzymschicht (3) an der Anode (1, 1a, 1b) und der Kathode (2, 2a, 2b) anliegt.

7. Elektrochemischer Sensor nach Anspruch 5 oder 6, **dadurch gekennzeichnet, dass** die Anode (1, 1a, 1b,) und die Kathode (2, 2a, 2b) auf einem gemeinsamen Träger (4) angeordnet sind und die Enzymschicht (3) sowohl die Anode (1, 1a, 1b) als auch die Kathode (2, 2a, 2b) bedeckt.

8. Elektrochemischer Sensor nach einem der Ansprüche 5 bis 7, **dadurch gekennzeichnet, dass** die Kathode (2, 2a, 2b) eine metallische Oberfläche hat.

9. Elektrochemischer Sensor nach einem der Ansprüche 5 bis 8, **dadurch gekennzeichnet, dass** die Anode (1, 1a, 1b) eine nicht-metallische Oberfläche hat.

10. Elektrochemischer Sensor nach einem der Ansprüche 5 bis 9, **dadurch gekennzeichnet, dass** das Enzym eine Oxidase, vorzugsweise eine Glucoseoxidase, ist.

11. Elektrochemischer Sensor nach einem der Ansprüche 5 bis 10, **dadurch gekennzeichnet, dass** das Produkt Wasserstoffperoxid ist.

12. Elektrochemischer Sensor nach einem der Ansprüche 5 bis 11, **dadurch gekennzeichnet, dass** die Enzymschicht (3) im trockenen Zustand ein Isolator ist.

13. Elektrochemischer Sensor nach einem der Ansprüche 5 bis 12, **dadurch gekennzeichnet, dass** ein zu der Anode (1, 1 a, 1 b) führender Leiter (5, 5a, 5b) über einen elektrischen Widerstand mit einem zu der Kathode (2, 2a, 2b) führenden Leiter (6, 6a, 6b) verbunden ist.

14. Elektrochemischer Sensor nach einem der Ansprüche 5 bis 13, **dadurch gekennzeichnet, dass** der Widerstand eine Mischung ist, die Kohlenstoffpartikel und Bindemittel enthält.

15. Elektrochemischer Sensor nach einem der Ansprüche 5 bis 14, **dadurch gekennzeichnet, dass** die Enzymschicht (3) mit einer Deckschicht (9) bedeckt ist.

## Claims

1. A method for the electrochemical measurement of an analyte concentration in vivo by means of a fuel cell which catalytically converts the analyte to be measured with an enzyme contained in an enzyme layer (3) and supplies an electrical voltage between an anode (1, 1 a, 1 b) and a cathode (2, 2a, 2b), said voltage being dependent on the analyte concentration and being measured, **characterized in that** by the catalytic conversion of the analyte a product is generated in the enzyme layer (3) which, as the fuel of the fuel cell, is oxidized on the anode (1, 1a, 1b) and is reduced on the cathode (2, 2a, 2b).

2. The method according to Claim 1, **characterized in that** the oxidation of the product is supported by a catalyst, preferably a metal oxide or a metallo-organic compound.

3. The method according to any one of the preceding claims, **characterized in that** the anode (1, 1 a, 1 b) and the cathode (2, 2a, 2b) are connected to each other via an electrical resistor and, as the measuring signal of the analyte concentration, the electrical voltage between the anode (1, 1a, 1b) and the cathode (2, 2a, 2b) is measured as a voltage drop at the resistor.

4. A fuel cell comprising an anode (1, 1 a, 1 b), a cathode (2, 2a, 2b) the surface of which is made of a different material than that of the anode (1, 1a, 1b), and an enzyme layer (3) which contains an enzyme for the catalytic conversion of a substance occurring in the human body, **characterized in that** the enzyme layer (3) forms a diffusion path to the anode and to the cathode for a product generated by catalytic conversion of the substance, with the result that the fuel cell oxidizes this product on the anode (1, 1a, 1b) and reduces it on the cathode (2, 2a, 2b).

5. An electrochemical sensor for the in vivo measurement of an analyte concentration by means of a fuel cell according to Claim 4, wherein the substance for the catalytic conversion of which the enzyme layer contains the enzyme is the analyte to be measured.

6. The electrochemical sensor according to Claim 5, **characterized in that** the enzyme layer (3) contacts the anode (1, 1a, 1 b) and the cathode (2, 2a, 2b).

7. The electrochemical sensor according to Claim 5 or 6, **characterized in that** the anode (1, 1 a, 1 b) and the cathode (2, 2a, 2b) are arranged on a common substrate (4) and the enzyme layer (3) covers both the anode (1, 1a, 1b) and the cathode (2, 2a, 2b).

8. The electrochemical sensor according to any one of Claims 5 to 7, **characterized in that** the cathode (2, 2a, 2b) has a metallic surface.

9. The electrochemical sensor according to any one of Claims 5 to 8, **characterized in that** the anode (1, 1 a, 1 b) has a non-metallic surface.

10. The electrochemical sensor according to any one of Claims 5 to 9, **characterized in that** the enzyme is an oxidase, preferably a glucose oxidase.

11. The electrochemical sensor according to any one of Claims 5 to 10, **characterized in that** the product is hydrogen peroxide.

12. The electrochemical sensor according to any one of Claims 5 to 11, **characterized in that** the enzyme layer (3) is an insulator in its dry state.

13. The electrochemical sensor according to any one of Claims 5 to 12, **characterized in that** a conductor (5, 5a, 5b) running to the anode (1, 1a, 1b) is connected to a conductor (6, 6a, 6b) running to the cathode (2, 2a, 2b) via an electrical resistor.

14. The electrochemical sensor according to any one of Claims 5 to 13, **characterized in that** the resistor is a mixture which contains carbon particles and a binding agent.

15. The electrochemical sensor according to any one of Claims 5 to 14, **characterized in that** the enzyme layer (3) is covered by a covering layer.

## Revendications

1. Procédé de mesure électrochimique d'une concentration en analyte *in vivo* utilisant une pile à combustible qui convertit de manière catalytique les analytes à mesurer grâce à un enzyme contenu dans une couche d'enzyme (3) et fournit une tension électrique, dépendante de la concentration en analyte à mesurer, entre une anode (1, 1a, 1b) et une cathode (2, 2a, 2b) et mesure celle-ci, **caractérisé en ce qu'**un produit qui est oxydé au niveau de l'anode (1, 1a, 1b) et est réduit au niveau de la cathode (2, 2a, 2b), en tant que combustible de la pile à combustible est créé dans la couche d'enzyme (3) lors de la conversion catalytique de l'analyte à mesurer.

2. Procédé selon la revendication 1, **caractérisé en ce que** l'oxydation du produit est entretenue avec un catalyseur, de manière préférée un oxyde métallique ou un composé organométallique.

3. Procédé selon l'une quelconque des revendications précédentes, **caractérisé en ce que** l'anode (1, 1a, 1b) et la cathode (2, 2a, 2b) sont reliées l'une à l'autre par l'intermédiaire d'une résistance électrique et la tension électrique entre l'anode (1, 1a, 1b) et la cathode (2, 2a, 2b) est mesurée au niveau de la résistance sous forme de chute de tension pour faire office de signal de mesure de la concentration en analyte.

4. Pile à combustible avec une anode (1, 1a, 1 b), une cathode (2, 2a, 2b), dont la surface est constituée d'un autre matériau que l'anode (1, 1 a, 1 b), et une couche d'enzyme (3) qui contient un enzyme destiné à la conversion catalytique d'une substance apparaissant dans le corps humain, **caractérisée en ce que** la couche d'enzyme (3) forme un chemin de diffusion vers l'anode et vers la cathode pour un produit créé par conversion catalytique de la substance, de sorte que la pile à combustible oxyde ledit produit au niveau de l'anode (1, 1a, 1b) et le réduit au niveau de la cathode (2, 2a, 2b).

5. Capteur électrochimique destiné à une mesure *in vivo* d'une concentration en analyte au moyen d'une pile à combustible selon la revendication 4, dans lequel la substance, pour la conversion catalytique de laquelle la couche d'enzyme contient un enzyme, constitue l'analyte à mesurer.

6. Capteur électrochimique selon la revendication 5, **caractérisé en ce que** la couche d'enzyme (3) repose sur l'anode (1, 1a, 1b) et la cathode (2, 2a, 2b).

7. Capteur électrochimique selon la revendication 5 ou 6, **caractérisé en ce que** l'anode (1, 1a, 1b) et la cathode (2, 2a, 2b) sont agencées sur un support commun (4) et la couche d'enzyme (3) recouvre aussi bien l'anode (1, 1 a, 1b) que la cathode (2, 2a, 2b).

8. Capteur électrochimique selon l'une quelconque des revendications 5 à 7, **caractérisé en ce que** la cathode (2, 2a, 2b) présente une surface métallique.

9. Capteur électrochimique selon l'une quelconque des revendications 5 à 8, **caractérisé en ce que** l'anode (1, 1 a, 1 b) présente une surface non métallique.

10. Capteur électrochimique selon l'une quelconque des revendications 5 à 9, **caractérisé en ce que** l'enzyme est une oxydase, de manière préférée une glucose oxydase.

11. Capteur électrochimique selon l'une quelconque des revendications 5 à 10, **caractérisé en ce que** le produit est du peroxyde d'hydrogène.

12. Capteur électrochimique selon l'une quelconque des revendications 5 à 11, **caractérisé en ce que** la couche d'enzyme (3) est un isolant à l'état sec.

13. Capteur électrochimique selon l'une quelconque des revendications 5 à 12, **caractérisé en ce qu'**un conducteur (5, 5a, 5b) menant à l'anode (1, 1a, 1b) est relié à un conducteur (6, 6a, 6b) menant à la cathode (2, 2a, 2b) par l'intermédiaire d'une résistance électrique.

14. Capteur électrochimique selon l'une quelconque des revendications 5 à 13, **caractérisé en ce que** la résistance est un matériau composite contenant des particules de carbone et un liant.

15. Capteur électrochimique selon l'une quelconque des revendications 5 à 14, **caractérisé en ce que** la couche d'enzyme (3) est recouverte d'une couche de revêtement (9).
